(19) Europäisches Patentamt
European Patent Office
Office européen des brevets

(11) Publication number: **0 123 736**
**A1**

(12) # EUROPEAN PATENT APPLICATION

(21) Application number: **83200618.3**

(22) Date of filing: **29.04.83**

(51) Int. Cl.³: **C 07 J 13/00**, C 07 J 41/00, C 07 J 43/00

(43) Date of publication of application: **07.11.84**
**Bulletin 84/45**

(84) Designated Contracting States: **NL**

(71) Applicant: **Gist - Brocades N.V., Wateringseweg 1 P.O. Box 1, NL-2600 MA Delft (NL)**

(72) Inventor: **Van Leusen, Albert Mattheus, De savornin Lohmanlaan 23, NL-9722 HC Groningen (NL)**
Inventor: **Van Leusen, Adriaan Marinus, Binnensingel 8, NL-9951 GD Winsum (Gr.) (NL)**

(74) Representative: **Huygens, Arthur Victor, Dr. et al, c/o Gist-Brocades N.V. Patent & Trademarks Department Wateringseweg 1 PO-box 1, NL-2600 MA Delft (NL)**

(54) **New process for the preparation of 21-hydroxy-20-keto-delta 16-steroids and new intermediate compounds formed in this process.**

(57) The invention relates to a new process for the preparation of 21-hydroxy-20-keto-steroids from 17-isocyanosulfonylmethylene-steroids whereby simultaneously an unsaturated bond is introduced between $C_{16}$ and $C_{17}$. Furthermore, the invention relates to new intermediate compounds formed in this process and their preparation.

EP 0 123 736 A1

## New process for the preparation of 21-hydroxy-20-keto-delta$^{16}$-steroids and new intermediate compounds formed in this process

The invention relates to a new process for the preparation of 21-hydroxy-20-keto-steroids from 17-isocyano-sulfonylmethylene-steroids whereby simultaneously an unsaturated bond is introduced between $C_{16}$ and $C_{17}$. Furthermore, the invention relates to new intermediate compounds formed in this process and their preparation.

More particularly, the invention relates to a new process for the introduction into steroids of a 17-side chain containing a 21-hydroxy group and a 20 keto group under the simultaneous formation of an unsaturated bond between $C_{16}$ and $C_{17}$ by reaction of 17-(isocyano-sulfonylmethylene)-steroids with an aldehyde and an alcohol to 17-(2-alkoxy-3-oxazolin-4-yl)-delta$^{16}$-steroids, and hydrolysis of these latter compounds to 21-hydroxy-20-keto-delta$^{16}$-steroids. The invention also relates to intermediate compounds which are formed in this process, i.e. 17-(2-alkoxy-3-oxazolin-4-yl)-delta$^{16}$-steroids and their preparation.

The preparation of 17-(isocyano-sulfonylmethylene) steroids is disclosed in the simultaneously filed application No. 00000000, entitled: "17-(Isocyano-sulfonylmethylene)-steroids, 17-(formamido-sulfonylmethylene)-steroids and their preparation", the contents of which have to be regarded as included herein.

Steroids are used on a large scale as constituents of many kind of pharmaceutical compositions. Dependant on the substituent patterns of the carbon-skeleton the steroids can be divided into a number of main classes. An important main-class of steroids is formed by the cortico-steroids. The natural representatives of the corticosteroids are usually produced by the adrenal gland. Corticosteroids are characterized by the presence of a 3-keto group, a delta$^{4}$ bond, an 11-bêta-hydroxy group, a 17-alpha-hydroxy group and a 17-bêta-hydroxy-acetyl side chain.

/

During a long time corticosteroids were made by chemical degradation of gall acids as cholic acid, desoxycholic acid and glycocholic acid. Afterwards, hecogenin, which could be isolated from plants, particularly from numerous Agave species, became an important raw material too. Since the possibility of introduction of an 11-hydroxy group by microbiological methods, diosgenin, which could be isolated from numerous Dioscoreaceae species, and stigmasterol, usually isolated from the phytosterol mixture from soya or calabar beans, have become the most important raw materials for the preparation of corticosteroids.

Much attention has been given to new, cheaper raw materials for the synthesis of pharmaceutical active steroids. Therefore, the degradation of the abundant soya bean derived sterols sitosterol and campesterol by microbiological methods into 17-oxo-steroids was extensively investigated. As a result thereof 17-oxo-steroids are readily available now against low prices, which makes these compounds, together with the possibility of the introduction of an 11-hydroxy group by microbiological methods, ideal starting materials for corticosteroid synthesis.

A number of chemical synthesis for the construction of the corticosteroid side chain from 17-oxo-steroids is known. For instance in Journal Org. Chem., 44, 1582 (1979) a method is described which uses a sulfenate-sulfoxide rearrangement for the introduction of the 17-(dihydroxyacetone) side chain. Another route is described in J.C.S. Chem. Comm., 1981, 775, in which the reaction of 17-oxo-steroids with ethyl isocyanoaetate is described, followed by a number of other reactions, which ultimately result in the dihydroxyacetone side chain of corticosteroids. Other syntheses of the corticosteroid side chain or of compounds which can be used as precursors therefore are described in J.C.S. Chem. Comm., 1981, 774, J.C.S. Chem. Comm. 1982, 551, Chem. Ber., 113, 1184 (1980), and J. Org. Chem., 1982, 2993.

In the abovementioned simultaneously filed application a process is described for the introduction of a 17-(isocyanosulfonylmethylene)-group into a 17-oxo-steroid. The present invention now concerns the reaction of the abovementioned 17-isocyano-sulfonylmethylene-steroids (I) with an aldehyde and an alcohol to 17-(2-alkoxy-3-oxazolin-4-yl)-delta$^{16}$-steroids (II), followed by hydrolyses of these latter compounds into 21-hydroxy-20-keto-delta$^{16}$-steroids (III).

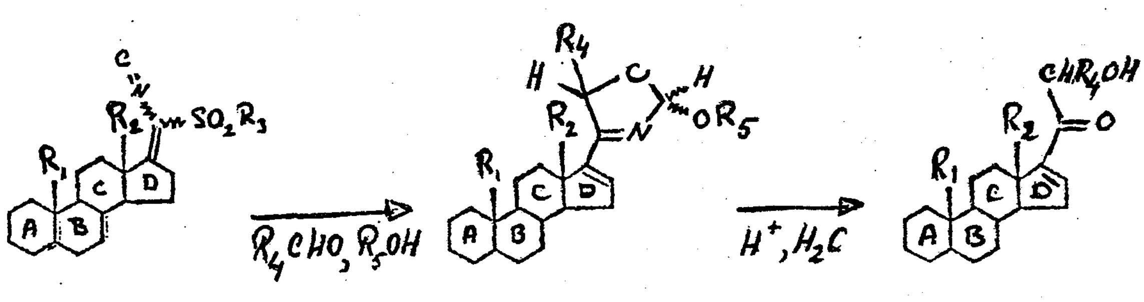


The process of the invention is characterized by reacting a 17-(isocyano-sulfonylmethylene)-steroid with an aldehyde and an alcohol, followed by hydrolysis.

Suitable 17-(isocyano-sulfonylmethylene-steroids for the process of the invention have the general formula

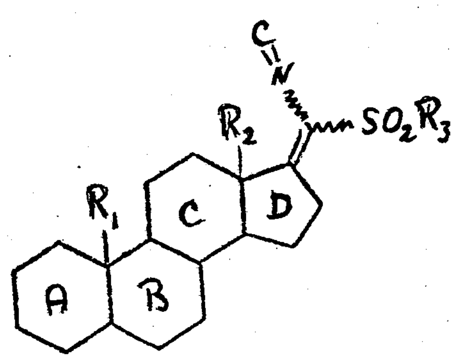

in which $R_1$ represents a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino or an aryl group substituted or not substituted by one or more halogen atoms, alkyl, alkoxy, nitro or cyano groups, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

When $R_3$ represents an alkyl group, suitable alkyl groups are straight or branched alkyl groups having 1 to 8 carbon atoms, preferably 1-4 carbon atoms.

When $R_3$ represents a dialkylamino group, suitable dialkylamino groups are dialkylamino groups wherein the alkyl groups are the same or different and contain 1-8 carbon atoms, preferably 1-4 carbon atoms, or a dialkylamino group wherein the alkyl groups together with the nitrogen atom form a heterocyclic ring which optionally my contain an oxygen atom, the ring containing up to 8 ring atoms. Prefered dialkylamino groups are dimethylamino, diethylamino, pyrolidine and morfoline.

When $R_3$ represents an aryl group, a suitable group is phenyl substituted or not substituted by a halogen atom or an alkyl group, preferably a phenyl or p-methylphenyl group.

When the rings A, B, C and D contain one or more double bonds, these double bonds are preferably present between $C_1$ and $C_2$, $C_3$ and $C_4$, $C_4$ and $C_5$, $C_5$ and $C_6$, $C_6$ and $C_7$, $C_9$ and $C_{10}$, $C_9$ and $C_{11}$ and/or $C_{11}$ and $C_{12}$. More preferably the double bond is present between $C_9$ and $C_{11}$.

When two or more double bonds are present especially the following systems are prefered: $C_3$-$C_4$ and $C_5$-$C_6$, $C_4$-$C_5$ and $C_6$-$C_7$, $C_1$-$C_2$ and $C_4$-$C_5$, $C_1$-$C_2$, $C_3$-$C_4$ and $C_5$-$C_{10}$ and $C_1$-$C_2$, $C_4$-$C_5$ and $C_6$-$C_7$. Preferably there is also a double bond between $C_9$ and $C_{11}$.

When the rings A, B, C and D are substituted by a hydroxy group, suitable groups are 3-, 9-, 11-, 12- or 14-hydroxy groups.

When the rings A, B, C and D are substituted by an amino group, suitable amino groups are 3-alkylamino groups, preferably containing 1-4 carbon atoms, 3-dialkylamino groups wherein the alkyl groups are the same or different, each alkyl group preferably containing 1-4 carbon atoms, or amino groups in which the nitrogen atom together with the alkyl group form a heterocyclic ring, preferably containing 1-8 ring atoms, which ring optionally may contain an oxygen atom. Particularly preferred are dimethylamino, diethylamino, pyrolidine an morfoline.

When the rings A, B, C and D are substituted by an oxygen atom this oxygen atom is preferably present at $C_3$, $C_{11}$ or $C_{12}$.

When the rings A, B, C and D are substituted by a halogen atom, suitable halogen atoms are 6-, 9- or 11-fluorine, chlorine or bromine atoms, preferably 6- or 9-fluorine or chlorine atoms.

When the rings A, B, C and D are substituted by an alkyl group, suitable alkyl groups are 1-, 2-, 6-, 7- or 16 methyl groups, preferably 1-, 6- and 16-methyl.

When the rings A, B, C and D are substituted by an alkoxy group, suitable alkoxy groups are 3-, 9-, 11- or 12-alkoxy groups containing 1-4 carbon atoms, preferably 3-, 9-, or 11-methoxy or ethoxy groups.

When the rings A, B, C and D are substituted by an alkoxyalkoxy group, suitable groups are 3- or 11-methoxymethoxy, methoxyethoxy or tetrahydropyranyloxy.

When the rings A, B, C and D are disubstituted, suitable substituents are epoxy groups at $C_1$ and $C_2$ or $C_9$ and $C_{11}$ or a methylene group attached to $C_1$ and $C_2$ or a 3,3-alkylenedioxy a 3,3-alkylenedithio or a 3,3-alkyleneoxythio group. The alkylene group preferably contains 2 or 3 carbon atoms.

More particularly the invention relates to compounds in which $R_1$ and $R_2$ represents methyl or in which $R_1$ is absent, which are substituted by halogen, especially fluorine, or hydroxy at $C_9$ and a hydroxy or keto group at $C_{11}$, or containing functional groups, as a double bond or epoxy group between $C_9$ and $C_{11}$, which can be converted by methods known in the art into the group mentioned before, and which contain a keto group at $C_3$ and double bonds between $C_1$ and $C_2$ and/or $C_4$ and $C_5$, or containing functional groups which can be converted in the keto group and double bonds mentioned before.

The reaction of the 17-(isocyano-sulfonylmethylene)-steroid with an aldehyde and an alcohol is carried out under basic conditions.

Suitable conditions are phase transfer-conditions, i.e. a two phase system of an organic layer and an aqueous layer to which an amount of a phase transfer catalyst salt has been added. Suitable organic solvents for the organic layer are methylenechloride, chloroform, 1,2- dichloroethane, benzene, toluene, chlorobenzene and dichlorobenzene. In general all those organic solvents can be used which are immiscible with water, and in which the relevant compounds are soluble. See for a general survey of phase-transfer reactions E.V. Dehmlov and S.S. Dehmlov,Phase Transfer Catalysis, Weinheim Chemie, 1980.

Suitable aqueous layers are solutions of alkali metal hydroxides in water, for example 5-50% solution of

lithium hydroxide, sodium hydroxide or potassium hydroxide. Suitable phase transfer catalysts are quaternary ammonium and fosfonium salts for instance, trimethylbenzyl ammonium halogenide, triethyl benzyl ammonium halogenide, tetrabutyl ammonium halogenide and alkyl triaryl fosfonium halogenides.

Examples of aldehydes wich can be used in the reaction are arylaldehydes, alkylaldehydes and formaldehyde. Suitable aldehydes are phenylaldehydes in which the phenyl group optionally is substituted by one or more halogen atoms or alkyl or alkoxy groups, alkyl aldehydes in which the alkyl group has 1-4 carbon atoms and optionally is substituted by one or more halogen atoms or alkoxy groups. Prefered aldehydes are benzaldehyde, acetaldehyde and formaldehyde, more particularly formaldehyde. The formaldehyde is used either as formaline (solution in water), as paraformaldehyde or as trioxan.

Suitable alcohols are $C_1$-$C_4$ alkyl alcohols and benzylalcohol. Preferably methanol is used.

The hydrolysis of the oxazolinyl compound to the 21-hydroxy-20-keto-delta$^{16}$-steroid can be carried out in an organic solvent, using an acidic aqueous solution. Suitable organics solvents are for istance diethyl ether, methanol and tetrahydrofuran. Suitable acids are diluted strong acids as hydrogen chloride, sulfuric acid and fosforic acid. Also acetic acid and formic acid can be used.

It is observed that sometimes not only the oxazolinyl group is hydrolyzed but also other groups linked to the steroid skeleton. These groups may have the function of protective groups (compare what is stated on protective groups in the simultaneously filed application entitled "17-(isocyano-sulfonylmethylen)-steroids and 17-(formamido-sulfonylmethylene)-steroids and their preparation."

The invention comprises also the 17-(2-alkoxy--3-oxazolin-4-yl)-delta$^{16}$-steroids and the 21-hydroxy-20-keto-delta$^{16}$-steroids as described above as far as these latter compound are novel.

The invention is elucidated by means of the following examples in which THF = tetrahydrofuran and Triton B = benzyltrimethylammonium hydroxide (40% solution in methanol).

Example 1a

Preparation of 3-methoxy-17-(2-methoxy-3-oxazolin-4-yl)androsta-3,5,16-triene.

3-Methoxy-17-(isocyano-p-methylphenylsulfonylmethylene)androsta-3,5-diene (239 mg, 0.5 mmol was dissolved in 10 ml benzene. To this solution were added formaline (0.2 ml 40% solution in water), methanol (0.2 ml), benzyl triethyl ammoniumchloride (15 mg) and 50% aqueous NaOH solution (4 ml). The mixture was stirred vigorously for 1 hour at 20°C. Extraction with benzene, filtration over $Al_2O_3$ (act. II-III; layer of 2 cm) followed by a washing with methylenechloride (50 ml) and evaporation in vacuo afforded the methoxyoxazoline (170 mg, 90%). IR (neat) 1655, 1630 $cm^{-1}$. $^1H$ NMR: ($CDCl_3$) delta 0.8-2.7 (m), 1.0 (s, 6H), 3.27 (s, 3H), 3.49 (s, 3H), 4.62 (m, 2H), 5.08, 5.18 (m, 2H), 6.2 (m, 1H), 6.45 (m, 1H).

Example 1b

Preparation of 21-hydroxypregna-4,16-diene-3,20-dione.

The above prepared methoxyoxazoline (170 mg, 0.45 mmole) was dissolved in a mixture of 8% $H_2SO_4$ (2.5 ml) and 10 ml THF. After standing at ambient temperature for 18 hours, water was added (10 ml). The THF was removed by evaporation in vacuo at 20°C, whereafter the precipated solid was collected, washed with water and dried. Yield: 149 mg (90%; calculated on the isocyanide) m.p. 215-220°C. (dec; acetone-/petether 40-60°C); (alpha)$^{20}$ +145° (c 1.0, $CHCl_3$); IR (Nujol) 3350 (OH), 1665 (C=O), 1620 (C=C). $^1H$ NMR ($CDCl_3$): delta 0.8-2.7 (m), 1.0 (s, 3H), 1.2 (s, 3H), 3.68 (s, 1H), 4.4 (s, 2H), 5.65 (s, 1H), 6.68 (m, 1H); Mass: $M^+$ 328 (calcd. 328); (Litt.: Allen and Bernstein, J. Amer. Chem. Soc. 77, 1028 (1975), m.p. 227-232°C).

Example 2

Preparation of 3-methoxy-21-hydroxypregna-1,3,5(10),16-tetraen-20-one.

To a solution of 3-methoxy-17-(isocyano-p-methylphenylsulfonylmethylene)estra-1,3,5(10)-triene (461 mg, 1 mmol) in benzene (20 ml) were added paraformaldehyde (300 mg, 10.0 mmol), methanol (0.4 ml), benzyltriethylammonium chloride (23 mg, 0.1 mmol) and 50% aqueous NaOH solution (8 ml). The mixture was stirred vigorously for one hour, then the organic solvent phase was separated from the water phase and the latter was once extracted with 5 ml of benzene. The combined benzene fractions were filtered over $Al_2O_3$ (act. II-III) followed by a washing with methylenechloride (100 ml). After evaporation of the solvent 3-methoxy-17-(2-methoxy-3-oxazolin-4-yl)estra-1,3,5(10),16-tetraene) was obtained as an oil. Without further processing this oxazolinyl compound was hydrolyzed by dissolving it in 15 ml THF and 5 ml 8% $H_2SO_4$. After 18 hours storage at room temperature the THF was evaporated in vacuum at 20°C. The remaining solid substance was collected, washed with water and dried. The yield was 300 mg (92%), m.p. 120-142°C. After two recrystallizations from ether/petether 40-60°C (1:4) the melting point of the product was 144-146°C. $(alpha)^{20}$ + 81 (c 1.00 $CHCl_3$). IR (Nujol) 3550 (OH), 1670 (C=O), 1620 (C=C), 1590 (Ar) $cm^{-1}$. $^1H$ NMR ($CDCl_3$): delta 0.9-3.6 (m), 0.95 (s), 3.71 (s, 3H), 4.42 (s, 2H), 6.53, 6.65, 6.71, 7.02, 7.12, (m, 4H). Anal. calcd. for $C_{21}H_{26}O_3$ (326.44): C 77.27, H 8.03; found: C 77.1, H 8.1.

Example 3

Preparation of 21-hydroxypregna-1,4,16-triene-3,20-dione.

17-(isocyano-p-methylphenylsulfonylmethylene)-androsta-1,4-dien-3-one (461 mg, 1.0 mmol) was converted to the hydroxyacetyl compound as described in example 2 (with the difference that during the first reaction step the benzene solution was stirred during half an hour). Yield 83% m.p. 155-168°C. After two crystallizations from ether the melting point of the product was 162-174°C. $(alpha)^{20}$ + 92.5 (c 1.00 $CHCl_3$). IR (Nujol): 3500 (OH), 1665 (C=O), 1630, 1610, 1590 (C=C) $cm^{-1}$. $^1H$ NMR ($CDCl_3$): 0.8-2.7 (m), 1.00 (s), 1.25 (s), 3.25 (br. 1H), 4.40 (s, 2H), 5.95-6.10 (m, 2H), 6.21, 6.23 (d, 1H), 6.6-6.7 (m, 1H). Anal. calcd. for $C_{21}H_{26}O_3$ (326.439): C 77.27, H 8.03; found C 76.7, H 8.1%.

The experiment was repeated under the same conditions, except the stirring time of the phase transfer reaction (first reaction step). Stirring for one hour resulted

in a yield of 70%, stirring for two hours resulted in a yield of 52%.

Example 4

Preparation of 21-hydroxypregna-4,9(11),16-triene-3,20-dione.

3-methoxy-17-(isocyano-p-methylphenylsulfonyl-methylene)-androsta-3,5,9(11)-triene (475 mg, 1.0 mmol) was converted to the hydroxyacetyl compound as described in example 2. Yield 300 mg, 93%, m.p. 180-193°C. After two crystallizations from methylenechloride/ether (2:7) the melting point of the product was 205-208°C., $(alpha)^{20}$ + 204° (c 1.00 $CHCl_3$). IR (Nujol): 3510 (OH), 1675 (C=O), 1620, 1595 (C=C), 1100 $cm^{-1}$. $^1H$ NMR ($CDCl_3$) delta 0.7-3.3 (m), 0.90 (s), 1.37 (s), 3.06 (s), 4.38 (s, 2H), 5.30-5.60 (m, 2H), 5.27 (s), 6.55-5.80 (m, 1H). (Litt.: J. Amer. Chem. Soc. 77, 1028 (1955); m.p. 204-209 and m.p. 215-218, $(alpha)^{20}$ + 194° (c 1.0, $CHCl_3$)).

Example 5

Preparation of 11-bêta-21-dihydroxypregna-4,16-diene-3,20-dione.

3-Methoxy-17-(isocyano-p-methylphenylsulfonyl-methylene)-11-bêta-hydroxyandrosta-3,5-diene (493 mg 1.0 mmol) was converted to the hydroxyacetyl compound as described in example 2 (with the difference that the stirring time during the first reaction step was 3/4 hour). Crude product 233 mg (68%). After crystallization from acetone-ether (1:3) m.p. 148°-153°C, $(alpha)^{20}$ + 198° (c 1.0, $CHCl_3$). IR (Nujol) 3500 (OH), 1690 (C=O), 1655 (br, C=C+C=O) $cm^{-1}$. 1H NMR ($CDCl_3$) delta 0.75-2.9 (m), 1.25 (s), 1.46 (s), 3.0-3.7 (m, 2H), 4.37 (br.s, 3H), 5.57 (s, 1H), 6.48-6.72 (m, 1H). Litt. J. Amer. Chem. Soc. 77, 1028 (1955); m.p. 154-156°C, $(alpha)^{20}$ + 200° (c 0.47, $CHCl_3$).

It is observed that the yield can be improved by a more thorough washing with $CH_2Cl_2$. $^1H$ NMR of the intermediate 3-methoxy-11-bêta-hydroxy-17-(2-methoxy-3-oxazolin-4-yl)androsta-3,5,16-triene:
($CDCl_3$): delta 0.7-2.9 (m), 1.23 (s, 3H), 1.28 (s, 3H), 3.27 (s, 3H), 3.48 (s, 3H), 4.25 (s, 1H), 4.61 (m, 2H), 5.00 (m, 2H), 6.13 (m, 1H), 6.40 (m, 1H).

Example 6

Preparation of 9alpha-fluoro-11bêta,21-dihydroxypregna-4,16-diene-3,20-dione.

3-Methoxy-9-alpha-fluoro-11-bêta-hydroxy-17-(isocyano-p-methylphenylsulfonylmethylene)androsta-3,5-diene was converted to the hydroxyacetyl compound as described in example 2. Yield 71%, m.p. after crystallization from $CH_2Cl_2$-petether 40-60°C (1:1), $(alpha)^{20}$ +165°C (c 0.9, $CHCl_3$). IR (Nujol) 3500 (OH), 1670 $cm^{-1}$ (br, C=O). $^1H$ NMR ($CDCl_3$) delta 0.7-3.7 (m), 1.23 (s), 1.57 (s), 4.17 (br.s, 1H), 4.40 (s, 2H), 5.70 (s, 1H), 6.55-6.80 (m, 1H).
Litt. US 2,963,496 discloses this compound without giving physical constants.

Example 7

Preparation of 21-hydroxypregna-4,16-diene-3,11,20-trione.

3-Methoxy-17-(isocyano-p-methylphenylsulfonyl-methylene)androsta-3,5-dien-11-one (491 mg, 1.0 mmol) was converted to the hydroxyacetyl compound as described in example 2 with the difference that the stirring time in the first reaction step was 15 minutes. Yield 250 mg (73%), m.p. after crystallization from $CH_2Cl_2$/ether 213-218°C, $(alpha)^{20}$ + 235° (c 1.00, $CHCl_3$). IR (Nujol), 3420 (OH), 1710, 1675 (C=O), 1625 (C=C) $cm^{-1}$. $^1H$ NMR ($CDCl_3$) delta 0.8-3.5 (m), 0.91 (s), 1.40 (s), 4.41, 4.48 (d, 2H), 5.68 (s, 1H), 6.64-6.83 (m, 1H). Litt. J. Amer. Chem. Soc. 77, 1028, (1955) m.p. 223-228°C., $(alpha)^{24}$ + 236° (cl, $CHCl_3$).

Example 8a

Preparation of 1-alpha,2-alpha-methylene-6-chloro-17-(2'-methoxy-3-oxazolin-4-yl)androsta-4,6,16-trien-3-on

1-Alpha,2-alpha-6-chloro-17-(isocyano-p-methylphenylsulfonylmethylene)androsta-4,6-dien-3-one (218 mg, 0.43 mmol) was dissolved in 8.6 ml benzene. 0.17 ml 40% formaline, 0.17 ml methanol, 0.03 ml Triton B and 3.44 ml aqueous NaOH solution (50%) were successively added to the benzene solution. The mixture was stirred at room temperature for half an hour. Then the two layers were separated and the water layer was washed two times with 5 ml benzene. The combined

benzene layers were filtered over $Al_2O_3$, followed by a washing with methylenechloride. After a further purification step by means of a chromatography with toluene, as well as increasing quantities of acetone 94 mg of the methoxyoxazoline compound was obtained, m.p. 164-167°C. IR 1655 (C(3)=O), 1630 (C=N), 1060 (COC) $cm^{-1}$. $^1H$ NMR ($CDCl_3$) delta 0.7-1.0 (m, cyclopropyl), 1.096 (s, 3H), 1.261 (s, 3H), 3.31 (s, 3H), 4.65 (m, 2H), 6.15 (m, 2H), 6.22 (m, 1H).

Example 8b

Preparation of 1-alpha,2-alpha-methylene-6-chloro-21-hydroxy-pregna-4,6,16-triene-3,20-dione.

The methoxyoxazolinyl compound prepared according to example 8a (90 mg) was dissolved in 8 ml THF, then 3 ml 2N $H_2SO_4$ was added. The mixture was stored at room temperature for 16 hours. After addition of $NaHCO_3$ (540 mg, 6 mmol) and some water the THF was removed by evaporation. The remaining solid substance was collected, washed with water and dried. Thus 57 mg of the hydroxyacetyl compound was obtained, m.p. 175-178°C. IR ($CHCl_3$) 1648, 1665 ($C^3$=O and $C^{20}$=O), 1588, 1608 (C=C) $cm^{-1}$. $^1H$ NMR ($CDCl_3$) delta 1.030 (s, 3H), 1.258 (s, 3H), 3.1 (s, br, 1H), 4.47 (s, 2H), 6.18 (m, 2H), 6.77 (m, 1H).

Example 9a

Preparation of 3,3-ethyleendithio-17-(2-methoxy-3-oxazolin-4-yl)-androsta-4,16-diene.

3,3-Ethyleendithio-17-(isocyano-p-methylphenyl-sulfonylmethylene)androsta-4-ene (540 mg, 1 mmol) was dissolved in 20 ml benzene. Then 0.4 ml formaline (40%), 0.4 ml methanol, 0.4 ml triton B and 8 ml 50 % aqueous NaOH solution were successively added. The mixture was stirred at room temperature during 20 minutes. After dilution with water the benzene layer was separated. The aqueous layer was extracted one time with 10 ml benzene. The collected benzene solutions were filtered over $Al_2O_3$ (act. II-III) and washed with

$CH_2Cl_2$. After evaporation of the solvent 0.5 g of the oxazolinyl compound was obtained. $^1H$ NMR ($CDCl_3$): delta 1.01 (s, 3H), 1.07 (s, 3H), 3.33 (m, 7H), 4.3-4.9 (m, 2H), 5.53 (s, 1H), 6.30 (s, 1H), 6.59 (t, 1H).

Example 9b

Preparation of 3,3-ethylenedithio-21-hydroxypregna-4,16-dien-20-one.

The oxazolinyl compound prepared according to example 9a (500 mg) was dissolved in 10 ml THF and 2.5 ml $H_2SO_4$ (8%). The mixture was stored at room temperature for 22 hours, then water (10 ml) was added and the THF removed by evaporation under reduced pressure. 280 mg of the hydroxyacetyl compound was obtained, m.p. 206-210°C (browning). IR ($CHCl_3$): 3475 (OH), 1665 (C=O), 1586 (delta$^{16}$) $cm^{-1}$. $^1H$ NMR ($CDCl_3$): delta 0.95 (s, 3H), 1.06 (s, 3H), 3.32 (s, 4H), 4.46 (s, 2H), 5.50 (s, 1H), 6.76 (t, 1H).

Example 10a

Preparation of 3,3-ethylenedioxy-17-(2'-methoxy-3-oxazolin-4-yl)-androsta-5,16-diene.

3,3-Ethylenedioxy-17-(isocyano-p-methylphenylsulfonylmethylene)androsta-5-ene (507 mg, 1 mmol) was dissolved 20 ml benzene. Then 0.4 ml formaline (40%), 0.4 ml methanol, 0.06 ml Triton B and 8 ml 50 % aqueous NaOH solution were added. The reaction mixture was stirred for half an hour at room temperature. Then the water layer was separated and washed two times with benzene. The combined benzene layers were filtered over $Al_2O_3$ (act. II-III), layer thickness 3.5 cm, and washed with $CH_2Cl_2$. The solvent was evaporated and thus 296 mg (72%) of the oxazolinyl compound, a white solid, was obtained: m.p. 154-157°C. IR ($CHCl_3$) 1623 (C=N), 1093 (C-O-C) $cm^{-1}$. $^1H$ NMR ($CDCl_3$) delta 1.017 (s, 3H) 1.077 (s, 3H), 3.29, 3.39 (2xs, 6H), 3.92 (s, 4H), 4.66 (m, 2H), 5.35 (m, 1H), 6.27 (m, 1H), 6.54 (t, 1H).

Example 10b

Preparation of 21-hydroxypregna-4,16-diene-3,20-dione.

The oxazolinyl compound prepared according to example 10a (222 mg) was dissolved in 15 ml THF, then 4.5 ml 2 N $H_2SO_4$ were added. The solution was kept for 16 hours at room temperature. The THF was evaporated and the remaining solid was washed with water. After drying under reduced pressure 178 mg substance were obtained. However, hydrolysis was not yet complete. Therefore a further reaction with 4 N $H_2SO_4$ in THF was applied, resulting into the pure hydroxy-acetyl compound (162 mg, 92%). IR and NMR were identically with the spectra of the product obtained in example 1b.

Example 11a

Preparation of 3-bêta-(2'-tetrahydropyranyloxy)-17-(2-methoxy-3-oxazolin-4-yl-)androsta-5,16-diene.

3-(2'-Bêta-tetrahydropyranyloxy)-17-(isocyano-p-methylphenylsulfonylmethylene)androsta-5-ene (570 mg, 1.04 mmol) was dissolved in 21 ml benzene, then formaline (0.42 ml), methanol (0.42 ml), Triton B (0.062 ml) and an aqueous NaOH solution (50%; 8.3 ml) were added. The reaction mixture was stirred at room temperature for 25 minutes. The benzene layer was separated and the water layer was washed with two portions of 5 ml benzene. The combined benzene solutions were filtered over $Al_2O_3$ (act. II-III) and washed with 100 ml $CH_2Cl_2$. After evaporation of the solvent the oxazolinyl compound was obtained (330 mg) IR ($CHCl_3$), 1623 (C=N), 1055 1025 (C-O-C). $^1H$ NMR ($CDCl_3$) delta 1.017 (s, 3H), 5.30 (m, 1H), 6.22 (m, 1H), 6.48 (m, 1H).

Example 11b

Preparation of 3,21-dihydroxypregna-5,16-dien-20-one.

The oxazolinyl compound prepared according to example 11a (152 mg, 0.27 mmol) was dissolved in 12 ml THF, then 3.5 ml 4 N $H_2SO_4$ was added. After storage at room temperature for 20 hours $NaHCO_3$ (1.359, 16 mmol) and 25 ml water were added. The THF was evaporated, the remaining solid collected, washed with water and dried. Column-purification using toluene-acetone (9:1) afforded 64 mg of the pure hydroxyacetyl compound; m.p. 194-197°C (browning at 187°C).

IR ($CHCl_3$) 3610, 3470 (OH), 1666 ($C^{20}$=O), 1585 (C=C) $cm^{-1}$. $^1H$ NMR ($CDCl_3$) delta 0.96 (s, 3H), 1.03 (s, 3H), 3.18 (s, 1H), 3.35 (m, 1H), 4.33 (m, 2H), 5.24 (m, 1H), 6.74 ($C^{16}H$).

Example 12

Preparation of 1alpha-methyl-3-methoxy-17-(2'methoxy-3-oxazolin-4-yl)androsta-3,5,16-triene.

The oxazolinyl compound was prepared as described in example 9a, starting form (982 mg, 2 mmol). Yield 653 mg (83 %). IR ($CHCl_3$) 1628 (C=N), 1057 (COC). NMR ($CDCl_3$) delta 0.78 (d, 3H9, 1.03 (s, 3H), 1.07 (s, 3H), 3.30 (s, 3H), 3.52 (s, 3H), 4.65 (m, 2H), 5.06 (m, 1H), 5.29 (m, 1H), 6.25 (m, 1H), 6.52 (m, 1H). In the same way as described in Example 9b the title compound can be hydrolized to a-alpha-hydroxypregna-4,16-diene-3,20-dione.

Example 12a.

Preparation of 3-methoxy-11-alpha-hydroxy-17-(2'methoxy-3-oxazolin-4-yl)androsta-3,5,16-triene.

To a solution of 3-methoxy-11-alpha-hydroxy-17-(isocyano-p-methylphenylsulfonylmethylene)androsta-3,5-diene (496 mg, 1 mmol) in toluene (20 ml) were added paraformaldehyde (300 mg, 10 mmol), methanol (0.8 ml) benzyltriethyl ammonium chloride (25 mg) and 50 % aqueous NaOH solution (8 ml). The mixture was stirred vigorously for 25 minutes. Extraction with toluene, filtration over $Al_2O_3$ (act. II-III), followed by a washing with methylen chloride and evaporation in vacuo afforded the oxazoline (196 mg). IR ($CHCl_3$) 1653, 1623 (C=C C=N) $cm^{-1}$. $^1$ H NMR ($CDCl_3$) delta 1.048 (s, 3H), 1.174 (s, 3H), 3.35 (s, 3H), 3.57 (s, 3H), 4.20 (m, 1H), 4.70 (m, 2H), 5.14 (s, 1H), 5.76 (m, 1H), 6.32 (tr, 1H), 6.57 (m, 1H).

Example 12 b

Preparation of 11-alpha, 21-dihydroxypregna-4,16-diene-3,20-dione.

The oxazolinyl compound prepared according to example 12a (98 mg) was dissolved in 5 ml THF, then 1.25 ml 4 N $H_2SO_4$ was added. After storage at room temperature for 20 hours 5 ml water was added. The THF was evaporated and the remaining substance was collected. Yield 69 mg. IR ($CHCl_3$), 3598 (OH), 3470 (OH), 1660 (C=O), 1615, 1589 (C=C) $cm^{-1}$. $^1H$ NMR ($CDCl_3$) delta 0.996 (s, 3H), 1.271 (s, 3H), 4.20 (m, 1H), 4.48 (s, 2H), 5.77 (s, 1H), 6.80 (tr. 1H).

Example 13 a

Preparation of 3-methoxy-6-chloro-17-(2-methoxy-3-oxazolin-4-yl)androsta-3,5,16-triene.

To a solution of 1 g 3-methoxy-6-chloro-17-(isocyano-p-methylphenylsulfonylmethylene)androsta-3,5-diene in 50 ml toluene were added 1 ml methanol, 1 ml formaline (49%), 75 mg benzyltriethl ammonium chloride and 20 ml aqueous NaOH solution (50%).The mixture was stirred vigorously for 1.5 hour at ambient temperature. Extraction with toluene, filtration over $Al_2O_3$ (act. II-III) followed by a washing with methylene chloride and evaporation afforded the oxazoline. IR ($CHCl_3$): 1643, 1619 (C=C + N=C) $cm^{-1}$. $^1H$ NMR ($CDCl_3$) delta 1.047 (s, 6H), 3.36 (s, 3H), 3.65 (s, 3H), 4.71 (m, 2H), 5.67 (s, 1H), 6.32 (tr, 1H), 6.58 (tr, 1H).

Example 13b

Preparation of 3-methoxy-6-chloro-21-hydroxypregna-3,5,16-trien-20-one.

The oxazolinyl compound prepared according to example 13a (400 mg) was dissolved in 20 ml THF, then 5 ml THF was added. After storage at ambient temperature for 18 hours 20 ml water was added. The THF was evaporated and the resulting substance was extracted with methylene chloride. The organic layer was dried and concentrated. Colomn-purification using toluene afforded 120 mg of the pure hydroxyacetyl compound; m.p. 158-160°C. IR ($CHCl_3$) 3471 (OH), 1670 (C=O) 1645, 1620, 1588 (C=C) $cm^{-1}$. NMR ($CDCl_3$) delta 0.983 (s, 3H), 1.044 (s, 3H), 3.65 (s, 3H), 4.48 (s, 2H), 5.66 (s, 1H), 6.78 (tr, 1H).

Example 14a

Preparation of 3-bêta-methoxymethoxy-17-(2'methoxy-3-oxazolin-4-yl)androsta-5,16-diene.

3-methoxymethoxy-17-(isocyano-p-methylphenylsulfonyl-methylene)androsta-5,16-diene (509 mg, 1mmol) was converted to the oxazolinyl compound as described in example 13a (with the difference that the mixture was stirred for 35 minutes). Yield 193 mg. $^1H$ NMR ($CDCl_3$) delta 0.99 (s, 3H), 1.04 (s, 3H), 3.33 (s, 7H), 4.65 (s, 4H), 5.35 (m, 1H), 6.35 (m, 1H), 6.53 (m, 1H).

Example 14b

Preparation of 3-bêta-methoxymethoxy-21-hydroxypregna-5,16-dien-20-one.

The oxazolinyl compound prepared according to example 14a (103 mg) was dissolved in 6 ml THF, then 1,5 ml 2N $H_2SO_4$ was added. After storage at room temperature for 48 hours 15 ml

water was added. The THF was removed by evaporation in vacuo, whereafter the precipated solid was collected, washed with water and dried. Yield 37 mg. $^1H$ NMR (CDCl$_3$) delta 0.95 (s, 3H), 1.05 (s, 3H), 3.34 (s, 4H), 4.42 (s, 2H), 4.64 (s, 2H), 5.35 (m, 1H), 6.71 (m, 1H).

Exmple 15a

Preparation of 17-(2'-methoxy-3-oxazolin-4-yl)androsta-4,16-dien-3-one

17-(isocyano-p-methylphenylsulfonylmethylene)androst-4-en-3-one (200 mg) was converted to the methoxyoxazolinyl compound as described in example 1a (with the difference that the mixture was stirred for 20 minutes). Yield 120 mg. $^1H$ NMR (CDCl$_3$) delta 0.99 (s, 3H), 1.18 (s, 3H), 3.28 (s, 3H), 4.64 (m, 2H), 5.68 (s, 1H), 6.23 (m, 1H), 6,49 (m, 1H).

Example 15b

Preparation of 21-hydroxypregna-4,16-diene-3,20-dione

The oxazolinyl compound prepared according to example 15a (120 mg) was converted to the hydroxyacetyl compound as described in example 14b. Yield 37 mg. IR and NMR were identically with the spectra of the products obtained in examples 1b and 10b.

Example 16

Preparation of 21-hydroxy-19-norpregna-4,16-diene-3,20-dione

To a solution of 351 mg 3-methoxy-17-(isocyano-p-methylphenylsulfonylmethylene)-19-norandrosta-3,5-diene in 15 ml toluene were added 0.3 ml formaline (40%), 0.3 ml methanol, 22 mg benzyltriethyl ammonium chloride and 6 ml aqueous NaOH solution (50%). The mixture was stirred vigorously for 65 minutes at ambient temperature. Extraction with toluene, filtration over $Al_2O_3$ (act. II-III) followed by a washing with methylene chloride and evaporation in vacuo afforded 112 mg 3-methoxy-17-(2methoxy-3-oxazolin-4'-yl)-19-norandrosta-3,5,16-triene. Without further processing this oxazolinyl compound was hydrolyzed by dissolving it in 8 ml THF and 2 ml $H_2SO_4$. After stirring for 17 houres at room temperature, 20 ml water was added. The THF was removed by evaporation in vacuo, whereafter the precipated solid was collected, washed with water and dried. Yield 58 mg. IR: ($CHCl_3$) 3475 (OH), 1666 (C=O), 1619, 1588 (C=C) $cm^{-1}$. $^1H$ NMR ($CDCl_3$): delta 1.02 (s, 3H), 3.24 (s, 1H), 4.48 (s, 2H), 5.87 (s, 1H), 6.78 (tr, 1H).

Example 17

Preparation of 1-alpha-methyl-3-methoxy-17-(2-methoxy-3-oxazolin-4-yl)androsta-3,5,16-triene.

The oxazolinyl compound was prepared as described in example 9a, starting from 1-alpha-methyl-3-methoxy-17-(isocyano-p-methylphenylsulfonylmethylene)-androsta-3,5-diene (982 mg, 2 mmol). Yield 653 mg (83 %). IR ($CHCl_3$) 1628 (C=N), 1057 (COC). $^1H$ NMR ($CDCl_3$) delta 0.78 (d, 3H), 1.03 (s, 3H), 1.07 (s, 3H), 3.30 (s, 3H), 3.52 (s, 3H), 4.65 (m, 2H), 5.06 (m, 1H), 5.29 (m, 1H), 6.25 (m, 1H), 6.52 (m, 1H). In the same way as described in Example 9b the title compound can be hydrolized to 1-alpha-methyl-21-hydroxypregna-4,16-diene-3,20-dione.

Claims

1. Process for the preparation of 21-hydroxy-20-keto-delta[16]-steroids characterized by reacting a 17-(isocyano-sulfonylmethylene)-steroid with an aldehyde and an alcohol, followed by hydrolysis.

2. Process for the preparation of 17-(2-alkoxy-3-oxazolin-4-yl)-delta[16]-steroids characterized by reacting a 17-(isocyano-sulfonyl-methylene)-steroid with an aldehyde and an alcohol.

3. Process for the preparation of 21-hydroxy-20-keto-delta[16]-steroids characterized by hydrolysis of 17-(2-alkoxy-3-oxazolin-4-y-)-delta[16]-steroids.

4. Process according to claims 1-3, characterized in that the 17-(isocyanosulfonylmethylene)-steroid has the general formula:

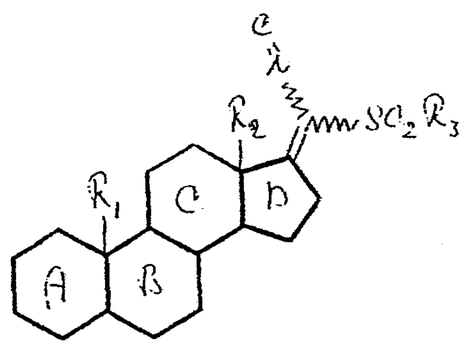

or the 17-(2-alkoxy-3-oxazolin-4-yl)-delta[16]-steroid has the general formula:

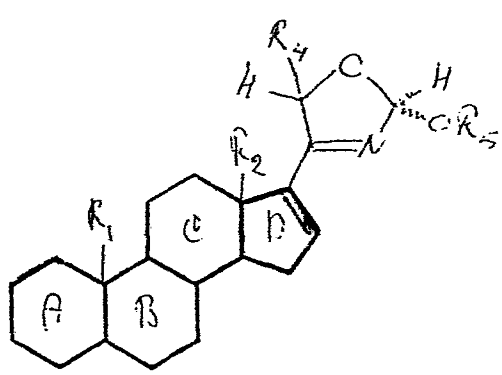

in which $R_1$ represents a hydrogen atom or a methyl group or is absent in the case of a double bond between $C_{10}$ adn $C_1$, $C_5$ or $C_9$, $R_2$ represents a hydrogen atom or a methyl group, $R_3$ represents an alkyl or dialkylaminogroup, or an aryl group substituted or not substituted by one or more halogen atoms, alkyl, alkoxy, nitro or cyano groups, $R_4$ represents a hydrogen atom or an alkyl or phenyl group, $R_5$ represents an alkyl group, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, alkylenedithio or alkyleneoxythio groups.

5. Process according to claims 1-4, characterized in that $R_4$ represents a hydrogen atom and $R_5$ represents a methyl group.

6. 17-(2-alkoxy-3-oxazolin-4-yl)-delta$^{16}$-steroids with the general formula:

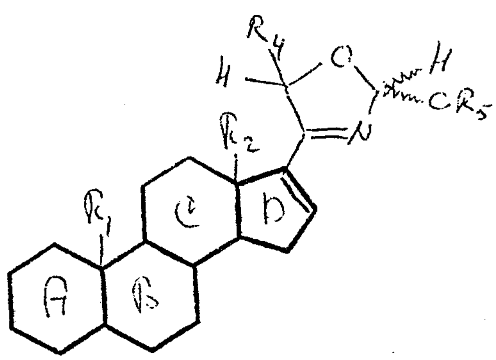

as defined in claim 4 and 5.

7. 21-Hydroxy-20-keto-delta$^{16}$-steroids with the general formula:

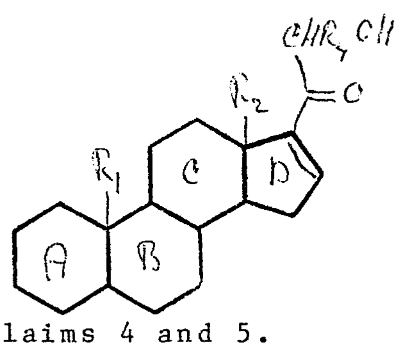

as defined in claims 4 and 5.

European Patent Office

**EUROPEAN SEARCH REPORT**

0123736

Application number

EP 83 20 0618

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-3 056 730 (ALLEN I. LASKIN) * Example 6 * --- | 7 | C 07 J 13/00<br>C 07 J 41/00<br>C 07 J 43/00 |
| X | CHEMICAL ABSTRACTS, vol. 91, no. 19, 5th November 1979, page 95, no. 151730t, Columbus, Ohio, USA A.V. KAMERNITSKII et al.: "Biological activity of transformed steroids. II. Relation between the degree of steroid molecule flatness and mineralocorticoid activity" & BIOORG. KHIM. 1979, 5(8), 1151-1157 * Abstract * --- | 7 | |
| X | DE-A-1 906 560 (SQUIBB) * Examples 7,8 * --- | 7 | TECHNICAL FIELDS SEARCHED (Int. Cl. 3) |
| X | FR-A-1 568 712 (UPJOHN) * Abstract points 10-14 * --- | 7 | |
| X | US-A-2 963 496 (GORDON H. THOMAS) * Claims * --- -/- | 7 | C 07 J 41/00<br>C 07 J 43/00<br>C 07 J 13/00 |

The present search report has been drawn up for all claims

| Place of search | Date of completion of the search | Examiner |
|---|---|---|
| THE HAGUE | 15-12-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0123736

Application number

EP 83 20 0618

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | JOURNAL OF THE AMERICAN CHEMICAL SOCIETY, vol. 77, no. 4, 28th February 1955, pages 1028-1032, Washington, D.C., USA W.S. ALLEN et al.: "Steroidal cyclic ketals. XII. The preparation of delta16-steroids" * Whole article * --- | 2 | |
| X | DE-C- 927 029 (FARBWERKE HOECHST AG) * Examples * --- | 7 | |
| X | FR-A-1 555 991 (SQUIBB) * Whole document * --- | 7 | |
| A | GB-A-2 086 907 (ROUSSEL UCLAF) * Whole document * --- | 1-6 | TECHNICAL FIELDS SEARCHED (Int. Cl. 3) |
| A | EP-A-0 058 097 (ROUSSEL UCLAF) * Claims * --- | 1-6 | |
| A | EP-A-0 023 856 (ROUSSEL UCLAF) * Claims * --- -/- | 1-6 | |

The present search report has been drawn up for all claims

| Place of search | Date of completion of the search | Examiner |
|---|---|---|
| THE HAGUE | 15-12-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0123736

Application number

EP 83 20 0618

Page 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | JOURNAL OF ORGANIC CHEMISTRY, vol. 44, no. 9, September 1979, pages 1582-1584, Washington, D.C., USA<br>VERLAN VanRHEENEN et al.: "New synthesis of Cortico steroids from 17-Keto steroids: Application and stereochemical study of the unsaturated sulfoxide-sulfenate rearrangement"<br>----- | 1-6 | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 15-12-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82